(19) Europäisches Patentamt
European Patent Office
Office européen des brevets

(11) Publication number: **0 415 598 A1**

# (12) EUROPEAN PATENT APPLICATION

(21) Application number: 90308960.5

(51) Int. Cl.[5]: A61K 7/06, A61K 7/48

(22) Date of filing: 15.08.90

(30) Priority: 16.08.89 GB 8918709

(43) Date of publication of application:
06.03.91 Bulletin 91/10

(84) Designated Contracting States:
AT BE CH DE DK ES FR GB GR IT LI NL SE

(71) Applicant: UNILEVER PLC
Unilever House Blackfriars
London EC4P 4BQ(GB)
(84) GB

Applicant: UNILEVER NV
Burgemeester s'Jacobplein 1 P.O. Box 760
NL-3000 DK Rotterdam(NL)
(84) BE

(72) Inventor: Brawn, Peter Rex,
Unilever Research Colworth Lab. Colworth House
Sharnbrook, Bedform MK44 1LQ(GB)

(74) Representative: Tonge, Robert James et al
UNILEVER PLC Patents Division P.O. Box 68
Unilever House
London EC4P 4BQ(GB)

## (54) Cosmetic composition.

(57) A composition suitable for topical application to mammalian skin and hair for inducing, maintaining or increasing hair growth comprises a hair growth promoter chosen from N-acylated amino-acids, in which the acyl group has from 2 to 20 carbon atoms, together with a cosmetically acceptable vehicle for the hair growth promoter.

EP 0 415 598 A1

# COSMETIC COMPOSITION

## FIELD OF THE INVENTION

The invention relates to cosmetic and pharmaceutical compositions for topical application to mammalian skin or hair, containing a hair growth promoter which is capable of increasing or maintaining hair growth, especially terminal hair growth on the human scalp.

## BACKGROUND

### The Hair Growth Cycle

It should be explained that in most mammals, hair does not grow continuously, but undergoes a cycle of activity involving alternate periods of growth and rest. The hair growth cycle can be divided into three main stages, namely:

(i) the growth phase known as anagen, during which the hair follicle penetrates deep into the dermis with the cells of the bulb dividing rapidly and differentiating to form the hair,

(i) the transitional stage known as catagen, which is heralded by the cessation of mitosis, and during which the follicle regresses upwards through the dermis and hair growth ceases,

(iii) the resting stage known as telogen, in which the regressed follicle contains a small secondary germ with an underlying ball of tightly packed dermal papilla cells.

The initiation of a new anagen phase is revealed by rapid proliferation in the germ, expansion of the dermal papilla and elaboration of basement membrane components. The hair cycle is then repeated many times until, as a consequence of the onset of male pattern baldness, most of the hair follicles spend an increasing proportion of their time in the telogen stage, and the hairs produced become finer, shorter, and less visible; this is known as terminal to vellus transformation.

## PRIOR ART

### Alleged Baldness Cures

Although there have been many claims in the scientific literature to the promotion or maintenance of hair growth by the topical application of hair tonics and the like, with the possible exception of minoxidil, none has been shown to be sufficiently free from disadvantageous clinical side effects, whether administered topically, orally or systemically, to warrant commercial exploitation as an ethical pharmaceutical, proprietary medicine, or as a cosmetic product. Possibly, the only means which has met with partial success for growing hair on the bald or balding human head is by transplantation of hair to the bald areas. This is, however, an extremely painful operation and is not always successful. Furthermore, it is immediately apparent to the casual observer that the subject has received a hair transplant and it may take many months or even years before hair regrowth, following this operation, assumes an appearance which resembles that of the original naturally growing hair.

Among the many hair regrowth studies that have been reported in the literature, there is included the work of Bazzano as described in PCT International Publication No. WO 85/04577. This publication describes a composition which is useful for increasing the rates of hair growth on mammalian skin, prolonging the anagen phase of the hair growth cycle and for treating various types of alopecias. The composition in question comprises a pyrimidine carbamate.

It has also been reported in US patent no. 4 139 619 to Chidsey assigned to the Upjohn Company, that a topical composition comprising minoxidil as the free base or acid addition salt thereof, or certain specified related iminopyrimidines, is useful in stimulating the conversion of vellus hair to growth as terminal hair, as well as increasing the rate of growth of terminal hair.

In spite of the apparent stimulation of hair growth or regrowth reported independently by Bazzano and Chidsey, following topical application of minoxidil or related compounds, there is general concern that systemic side-effects can result, particularly following topical application of minoxidil. Thus it is generally

recognised in the medical literature that the side effects of orally administered minoxidil are very serious, and include fluid retention, tachycardia, dyspnea, gynecomastia, fatigue, nausea and cardiotoxicity. There is also evidence that certain side effects have been experienced following topical application of minoxidil.

## BACKGROUND TO THE INVENTION

Our own search for effective compositions that could be applied topically to the human scalp in order to promote hair growth, was influenced by the need to discover molecules which were not only effective but also completely safe in use and free from contra indications which would limit their appeal. Furthermore, we were anxious to identify relatively simple molecules in this respect which were easy to synthesis and inexpensive to deploy in a mass market affordable product which would appeal to a large number of potential consumers.

It was accordingly during in vivo studies into the effects on rat skin of N-acetyl glycine, that it was observed unexpectedly that this substance may be capable of promoting hair growth. This was tested and evidence obtained to substantiate this surprising observation.

## DEFINITION OF THE INVENTION

Accordingly, the invention provides a preserved composition suitable for topical application to mammalian skin or hair for inducing, maintaining or increasing hair growth, which comprises:

i. an effective amount of from 0.001 to 99% by weight of a hair growth promoter chosen from mono N-acylated neutral or acidic amino acids or salts thereof, in which the acyl group has from 1 to 18 carbon atoms; and

ii. from 1 to 99.999% by weight of a cosmetically acceptable vehicle for the hair growth promoter; the total amount of the hair growth promoter present in the composition being sufficient to increase hair growth in the rat, when said composition is applied topically thereto over a period of no more than 3 months, by at least 10% more than that obtainable using a control composition from which the said promoter has been omitted, in accordance with the Rat Hair Growth Test.

## DISCLOSURE OF THE INVENTION

### The hair growth promoter

According to the invention, the composition comprises as a hair growth promoter, a mono N-acylated neutral or acidic amino acid, in which the acyl group has from 1 to 18, in which the acyl group has from 1 to 18 carbon atoms.

Preferably the acyl group is chosen from acetyl, hexanoyl, octanoyl, lauroyl, myristoyl, palmitoyl and stearoyl. The most preferred acyl group in acetyl.

Examples of the amino acid moiety from which the mono N-acylated neutral amino acid is derived include:

glycine
alanine
valine
leucine
isoleucine
phenylalanine
tyrosine
proline
hydroxyproline
serine
threonine
cysteine
cystine
methionine, and

tryptophan.

Preferred mono N-acylated neutral amino acids include:

N-acetyl glycine.
N-acetyl hydroxyproline
N-acetyl alanine
N-acetyl valine
N-acetyl leucine
N-acetyl isoleucine
N-acetyl phenylalanine
N-acetyl tyrosine
N-acetyl proline
N-acetyl serine
N-acetyl threonine
N-acetyl cysteine
N-acetyl cysteine
N-acetyl methionine
N-acetyl tryptophan
N-acetyl aspartic acid
N-acetyl glutamic acid
N-lauroyl glycine
N-palmitoyl glycine
N-myristoyl glycine>
N-lauroyl hydroxyproline
N-palmitoyl aspartic acid
N-octanoyl glycine
N-octanoyl hydroxyproline
N-hexanoyl glycine, and N-hexanoyl aspartic acid.

Examples of the amino acid moiety from which the mono N-acylated acidic amino acid is derived include:

aspartic acid and glutamic acid.

The mono N-acylated amino acids as herein defined can also be employed in the composition according to the invention as their corresponding salts, examples of which includes alkali metal salts, such as sodium and potassium and alkanolamine salts such as triethanolamine.

The composition can comprise two more mono N-acylated amino acids, or salts thereof.

The total amount of the hair growth promoter present in the composition according to the invention is sufficient to increase hair growth in the rat, the model selected for this test, when said composition is applied topically thereto over a period of no more than 3 months, by at least 10% more than that obtainable using a control composition from which the said promoter has been omitted, in accordance with the Rat Hair Growth Test.

Preferably, the amount of promoter should be sufficient to increase hair growth in the rat by at least 20%, more preferably by at least 30%, most preferably by at least 40% and ideally by at least 50%.

The effective amount which is sufficient to induce, maintain or increase hair growth will depend on the effectiveness of the promoter some being more effective than others, but in general, an amount of from 0.0001 to 99%, preferably from 0.01 to 20% by weight of the composition will provide an adequate dose to the skin after topical application.

## Preservation of the Composition

The composition according to the invention is preferably preserved in such a manner that it will enjoy an extended shelf life following manufacture and prior to sale and use. Ideally the composition will have an indefinite shelf life.

It is accordingly apparent that the hair growth promoter is likely to be prone to attack by bacteria, moulds and fungi and other microbial influences, particularly at pH values near that of the skin that characterise the preferred composition. The shelf-life of the composition can therefore be unacceptably short due to the biodegradation of the hair growth promoter unless steps are taken to preserve the composition.

In order to be preserved, the composition should preferably be free, or substantially free, from viable

microbial contaminants that are capable of resulting in microbial spoilage of the composition, and/or biodegradation of the hair growth promoter prior to topical application of the composition to mammalian skin or hair. It is to be understood, however, that the invention is also concerned with compositions, as herein defined, which may contain viable but dormant microorganisms, such as bacterial spores, provided that the conditions of preservation do not result in substantial proliferation of the microorganisms prior to use of the composition.

Examples of methods that can be employed to achieve preservation of the composition, includes the following:

(i) Sterilisation

The composition according to the invention can be preserved by sterilisation to remove or kill substantially all viable microbial contaminants. This can be achieved for example by irradiation using a lethal dose of gamma rays, by heat sterilisation or by ultrafiltration using techniques that are well established in the pharmaceutical industry.

(ii) Chemical Preservative

The composition according to the invention can also be preserved by including in it a chemical preservative which functions to prevent the growth of or kill bacteria, fungi or other microorganisms.

Examples of chemical preservatives include ethanol, benzoic acid, sodium benzoate, sorbic acid, potassium sorbate, sodium propionate and the methyl, ethyl, propyl and butyl esters of p-hydroxybenzoic acid. The amount of chemical preservative that can be incorporated in the composition according to the invention will generally be from 0.05 to 5%, preferably from 0.1 to 2% by weight, the amount chosen being sufficient to arrest microbial proliferation.

(iii) Water activity depressants

The composition according to the invention can also be preserved by the inclusion of a water activity depressant such as glycerol, propylene glycol, sorbitol, sugars and salts, for examples alkali metal halides, sulphates and carboxylates. When employing a water activity depressant, sufficient should be incorporated in the composition according to the invention to reduce the water activity ($\alpha_w$) from 1 to $< 0.9$, preferably to $< 0.85$ and most preferably $< 0.8$, the lowest of these values being that at which yeasts, moulds and fungi will not proliferate.

pH

The hair growth promoter may be susceptable to hydrolysis, particularly when the pH value of the composition is alkaline. It is accordingly preferred that the composition, when aqueous, should have an acid pH value. The preferred pH value of the composition, when aqueous, is from 2 to <7, ideally from 4 to 6.5.

The Vehicle

The composition according to the invention also comprises a solid, semi-solid or liquid cosmetically and/or physiologically acceptable vehicle, to enable the hair growth promoter to be conveyed to the skin at an appropriate dilution. The nature of the vehicle will depend upon the method chosen for topical administration of the composition. The vehicle can itself be inert or it can possess physiological or pharmaceutical benefits of its own.

The selection of a vehicle for this purpose presents a wide range of possibilities depending on the required product form of the composition. Suitable vehicles can be classified as described hereinafter.

It should be explained that vehicles are substances which can act as diluents, dispersants, or solvents for the hair growth promoter which therefore ensure that they can be applied to and distributed evenly over the hair and/or scalp at an appropriate concentration. The vehicle is preferably one which can aid

penetration of the esters into the skin to reach the immediate environment of the hair follicle. Compositions according to this invention can include water as a vehicle, and/or at least one cosmetically acceptable vehicle other than water.

Vehicles other than water that can be used in compositions according to the invention can include solids or liquids such as emollients, solvents, humectants, thickeners and powders. Examples of each of these types of vehicles, which can be used singly or as mixtures of one or more vehicles, are as follows:

Emollients, such as stearyl alcohol, glyceryl monoricinoleate, glyceryl monostearate, propane-1,2-diol, butane-1,3-diol, mink oil, cetyl alcohol, ispropyl isostearate, stearic acid, isobutyl palmitate, isocetyl stearate, oleyl alcohol, isopropyl laurate, hexyl laurate, decyl oleate, octadecan-2-ol, isocetyl alcohol, cetyl palmitate, dimethylpolysiloxane, di-n-butyl sebacate, isopropyl myristate, isopropyl palmitate, isopropyl stearate, butyl stearate, polythylene glycol, triethylene glycol, lanolin, sesame oil, coconut oil, arachis oil, castor oil, acetylated lanolin alcohols, petroleum, mineral oil, butyl myristate, isostearic acid, palmitic acid, isopropyl linoleate, lauryl lactate, myristyl lactate, decyl oleate, myristyl myristate;

Propellants, such as trichlorofluoromethane, dichlorodifluoromethane, dichlorotetrafluoroethane, monochlorodifluoromethane, trichlorotrifluoroethane, propane, butane, isobutane, dimethyl ether, carbon dioxide, nitrous oxide;

Solvents, such as ethyl alcohol, methylene chloride, isopropanol, castor oil, ethylene glycol monoethyl ether, diethylene glycol monobutyl ether, diethylene glycol monoethyl ether, dimethyl sulphoxide, dimethyl formamide, tetrahydrofuran;

Humectants, such as glycerin, sorbitol, sodium 2-pyrrolidone-5-carboxylate, soluble collagen, dibutyl phthalate, gelatin;

Powders, such as chalk, talc, fullers earth, kaolin, starch, gums, colloidal silicon dioxide, sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The amount of vehicle in the composition, including water if present, should preferably be sufficient to carry at least a portion of a selected ester to the skin in an amount which is sufficient effectively to enhance hair growth. The amount of the vehicle can comprise the balance of the composition, particularly where little or no other ingredients are present in the composition. Accordingly, the vehicle or vehicles can comprise from 1 to 99.99%, preferably from 50 to 99.5% and ideally from 90 to 99% by weight of the composition.

Perfume

The composition according to the invention can also optionally comprise a perfume in an amount sufficient to make the composition acceptable to the consumer and pleasant to use. Usually, the perfume will form from 0.01 to 10% by weight of the composition.

Activity Enhancer

The composition according to the invention can also optionally comprise an activity enhancer.

The activity enhancer can be chosen from a wide variety of molecules which can function in different ways to enhance the hair growth effects of the hair growth promoter. Particular classes of activity enhancers include other hair growth stimulants, penetration enhancers and cationic polymers, whose presence can further improve the delivery of the ester through the stratum corneum to its site of action in the immediate environment of the hair follicle.

Some activity enhancers can also function as vehicles for the ester.

(a) Other Hair Growth Stimulants

Examples of other substances which themselves possess the ability to stimulate or increase hair growth include, for example:

Benzalkonium chloride

Benzethonium chloride

Phenol

Estradiol
Diphenhydramine hydrochloride
Chlorpheniramine maleate
Chlorophyllin derivatives
Cholesterol
Salicylic acid
Cystine
Red pepper tincture
Benzyl nicotinate
dl-Menthol
Peppermint oil
Calcium pantothenate
Panthenol
Castor oil
Hinokitiol
Prednisolone
Resorcinol

Further substances which themselves possess the ability to increase the rate of terminal hair growth include:

α-1,4 esterified disaccharides described by Choay S.A. in EP-A-O 064 012, having the structure (2):

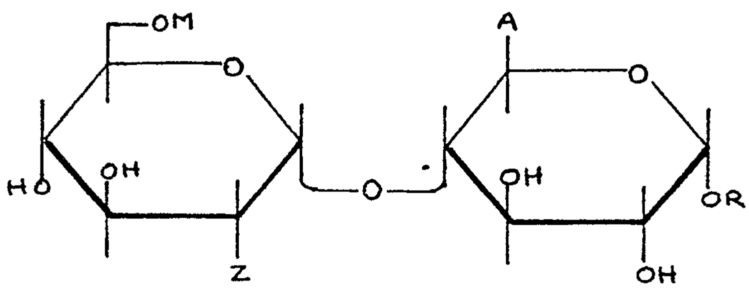

(2)

where

Z represents a functional nitrogen group, such as an azide or a group having the structure -NHB, in which B represents -H or a functional group such as acetyl or sulphate as a salt with an organic or mineral cation;

M represents -H or $SO_3M_1$, where $M_1$ is an organic or metallic cation, particularly an alkali metal; or an acetyl group;

R represents a $C_1$ to $C_4$ alkyl radical, especially methyl; or an aryl radical;

A represents a functional group such as an acid or $-COOR_1$, where $R_1$ represents -H or a $C_1$ to $C_4$ alkyl radical, especially methyl; or a metal, especially an alkali metal;

esterified oligosaccharides as described by Unilever in EP-A-O 211 610, including at least one esterified disaccharide unit consisting of a uronic acid residue having the structure (3):

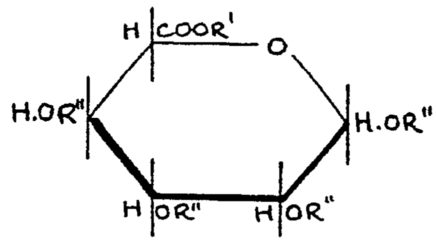

(3)

and a hexosamine residue having the structure (4):

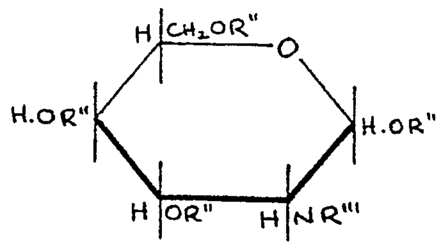

(4)

where
R' is -H, $C_3$ to $C_{10}$ alkyl or

$$\begin{array}{l} COOR'' \\ -CH(CH_2)_nCH_3 \end{array}$$

R'' is -H, $C_1$ to $C_4$ alkyl, $-CO(CH_2)_mCH_3$, $-SO_3M$,
R''' is -H, $-CO(CH_2)_mCH_3$, or $-SO_3M$,
M is -H, or a metallic or organic cation
n is 0 or an integer of from 1 to 7, and
m is 0 or the integer 1 or 2;
the groups designated R'' being the same or different, one R'' group from each pyranose ring structure being linked by a glycosidic linkage having the configuration α-1,3, α-1,4, β-1,3 or β-1,4; and the -COOR', $-CH_2OR''$ and -OR'' groups being of either configuration with respect to the pyranose rings;
Minoxidil glucuronides, as described by Unilever in EP-O 242 967,
Minoxidil sulphates, as described by The Upjohn Co. in WO 86/04231, and
Minoxidil, and other derivatives thereof as described by The Upjohn Co, in US patent 4 139 619.

Particularly preferred mixtures of minoxidil and a hair growth promoter according to the invention include the following:
Minoxidil and N-acetyl glycine
Minoxidil and N-acetyl hydroxyproline
Minoxidil and N-acetyl aspartic acid.

(b) Penetration Enhancers

As has been stated earlier, the presence of a penetration enhancer can potentiate the benefit of the hair growth promoter by improving its delivery through the stratum corneum to its site of action in the immediate environment of the hair follicle close to the dermal papilla.

The penetration enhancer can accordingly function in a variety of ways. It can for example, improve the distribution of the hair growth promoter on the skin surface or, it can increase its partition into the skin from

the composition when applied topically, so aiding its passage to its site of action. Other mechanisms enhancing the benefit of the hair growth promoter may also be involved.

Examples of penetration enhancers include:

2-methyl propan-2-ol
Propan-2-ol
Ethyl-2-hydroxypropanoate
Hexan-2,5-diol
POE(2) ethyl ether
Di(2-hydroxypropyl) ether
Pentan-2,4-diol
Acetone
POE(2) methyl ether
2-hydroxypropionic acid
2-hydroxyoctanoic acid
Propan-1-ol
1,4 Dioxane
Tetrahydrofuran
Butan-1,4-diol
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Debenzyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl palmitate
Ethyl laurate
2-ethyl-hexyl pelargonate
Isopropyl isostearate
Butyl laurate
Benzyl benzoate
Butyl benzoate
Hexyl laurate
Ethyl caprate
Ethyl caprylate
Butyl stearate
Benzyl salicylate
2-hydroxypropanoic acid

2-hyroxyoctanoic acid,

Further examples of penetration enhancers include:-

Dimethyl sulphoxide
N,N-Dimethyl acetamide
N,N-Dimethyl formamide
2-Pyrrolidone
1-Methyl-2-pyrrolidone
5-Methyl-2-pyrrolidone
1,5-Dimethyl-2-pyrrolidone
1-Ethyl 2-pyrrolidone
Phosphine oxides
Sugar esters
Tetrahydrofurfural alcohol
Urea
Diethyl-m-toluamide, and
1-Dodecylazacyloheptan-2-one

Further examples of penetration enhancers include surface active agents, preferred examples of which include:

(i) Anionic surface active agents, such as metallic or alkanolamine salts of fatty acids for example sodium laurate and triethanolamine oleate;
alkyl benzene sulphonates, for example triethanolamine dodecyl benzene sulphonate;
alkyl sulphates, for example sodium lauryl sulphate;
alkyl ether sulphates, for example sodium lauryl ether sulphate [2 to 8 EO];
sulphosuccinates, for example sodium dioctyl sulphosuccinate;
monoglyceride sulphates, for example sodium glyceryl monostearate monosulphate;
isethionates, for example sodium isethionate;
methyl taurides, for example Igepon T;
acylsarcosinates, for example sodium myristyl sarcosinate;
acyl peptides, for example Maypons and Lamepons;
acyl lactylates,
polyalkoxylated ether glycollates, for example trideceth-7 carboxylic acid;
phosphates, for example sodium dilauryl phosphate.

(ii) Cationic surface active agents, such as amine salts, for example sapamin hydrochloride;
quartenary ammonium salts, for example Quaternium 5, Quaternium 31 and Quaternium 18;

(iii) Amphoteric suface active agents, such as imidazol compounds, for example Miranol;
N-alkyl amino acids, such as sodium cocaminopropionate and asparagine derivatives;
betaines, for example cocoamidopropylbetaine

(iv) Nonionic surface active agents, such as fatty acid alkanolamides, for example oleic ethanolamide;
esters of polyalcohols, for example Span;
polyglycerol esters, for example that esterified with $C_{12-18}$ fatty acids and one or several OH groups;
polyalkoxylated derivatives, for example polyoxy:polyoxyethylene stearate, and octylphenoxy polyethoxyethanol (TRITON X-100);
ethers, for example polyoxyethylene lauryl ether;
ester ethers, for example Tween;
amine oxides, for example coconut and dodecyl dimethyl amine oxides.

Mixtures of two or more of the above surface active agents can be employed in the composition according to the invention.

(c) cationic polymers chosen from:

Guar Hydroxypropyltrimonium chloride
Quaternium-19
Quaternium-23
Quaternium-40
Quaternium-57
Poly(dipropyldiallylammonium chloride)
Poly(methyl-β-propaniodiallylammonium chloride)
Poly(diallylpiperidinium chloride)
Poly(vinyl pyridinium chloride)

Quaternised poly (vinyl alcohol)
Quaternised poly
(dimethylaminoethylmethacrylate); and
mixtures thereof

The amount of activity enhancer, when employed in accordance with the invention, will normally be from 0.1 to 50%, preferably from 0.5 to 25% and most preferably from 0.5 to 10% by weight of the composition.

Other hair growth promoter adjuncts

The composition according to the invention can also contain adjuncts other than those already mentioned, depending on the form of the intended product. It is, for example, possible to include antiseptics, preservatives, antioxidants, emulsifiers and colouring agents, which can improve the stability and consumer appeal of the composition.

The composition according to the invention can also be employed as a vehicle for a wide variety of cosmetically or pharmaceutically active ingredients, particularly ingredients which have some beneficial effect other than the promotion of hair growth when applied to the skin.

Process

The invention also provides a process for the preparation of a composition suitable for topical application to mammalian skin or hair which comprises mixing a hair growth promoter as herein defined, with a suitable vehicle to provide a composition according to the invention, in which the hair growth promoter forms from 0.0001 to 99% by weight of the composition.

Product Form and Container

The compositions of the invention can be formulated as liquids, for example as a lotion, shampoo, milk or cream for use in conjunction with an applicator such as a roll-ball applicator, or a spray device such as an aerosol can containing propellant, or a container fitted with a pump to dispense the liquid product. Alternatively, the compositions of the invention can be solid or semi-solid, for example sticks, creams or gels, for use in conjunction with a suitable applicator or simply a tube, bottle or lidded jar, or as a liquid-impregnated fabric, such as a tissue wipe.

The invention accordingly also provides a closed container containing a composition as herein defined.

Use of the hair growth promoter for Inducing, Maintaining or Increasing Hair Growth

The invention also provides for the use of hair growth promoter as herein defined, for topical application to mammalian skin or hair for inducing, maintaining or increasing hair growth.

The compositions according to the invention are primarily intended for topical application to the scalp of the human subject, particularly where the head is already bald or balding, in order to promote the regrowth of terminal hair. The compositions can also be applied profilactically to the hair and hence the scalp to reduce or prevent the onset of baldness.

The amount of the composition and the frequency of application to the hair and/or scalp can vary widely, depending on personal needs, but it is suggested as an example that topical application of from 0.1 to 5g daily containing from 0.00001 to 1g of a selected chemical inhibitor over the period of at least six months will in most cases result in an improvement in hair growth.

E VALUATION OF EFFICACY OF THE HAIR GROWTH PROMOTERS USING THE RAT MODEL

The Rat Hair Growth Test

The effect of compounds on hair growth was assessed using male albino Wistar rats as an animal model. The rats were chosen from as few litters as possible and were each approximately 42 days of age at the start of the test. Each rat was housed individually to prevent licking.

In each comparison, 10 rats were used in each group and hair growth was assessed as follows:

A small patch of normal skin (4cm x 4cm) on the upper back of each rat was clipped at the start and 0.3 ml of a hair growth stimulant composition (or a control) applied topically twice daily and once on Saturdays and Sundays to each clipped area. The concentration of test compound in the composition was chosen from 0.01 to 20% by weight.

It is to be understood that the potency of each of the hair growth promoters in terms of its ability to induce, maintain or increase hair growth is unlikely to be uniform, some being more potent than others, and therefore the concentration of any promoter chosen for thorough evaluation must be carefully selected after preliminary testing to determine its potential as a hair growth promoter. In any case, this concentration will lie within the range of from 0.01 to 20% by weight as stipulated above.

Hair was clipped from the area of the patch twice weekly, collected and weighed at each time point over a standard period of 3 months, and cumulative hair weight calculated. From these data, it was possible to estimate the effect of a hair growth promoter acid as a hair growth stimulant (test compound) on the amount and duration of hair growth during the experiment. A positive response, i.e. an increase of at least 10% by weight of hair after 3 months treatment, compared with a control indicates the potential of the test compound to prevent hair loss and/or reverse baldness in human subjects.

Accordingly, when the hair growth promoters as herein defined, are assessed either individually or in combination as test compound by the Rat Hair Growth Test, an increase of at least 10% by weight of hair after 3 months treatement will be obtained. Usually, the 10% by weight minimum value will be attained well before the end of this 3 months period.

Measurement of hair growth following topical application of N-acetyl glycine

Topical treatment with a composition according to the invention was found to stimulate hair growth. In this example, the effect of topical application of N-acetyl glycine is shown. The test solution in this experiment contained 10% (w/v) of the N-acetyl glycine in the form of a solution in distilled water adjusted to pH 4.2 with potassium hydroxide. The hair growth results are shown below in Table 1.

Table 1

| Treatment | Mean Cumulative Hair Weight (mg) ± sd, after 58 days | Significance Level (vs control) |
|---|---|---|
| 10% (w/v) N-acetyl glycine | 622.8 ± 101.11 | p |
| Control (dis. water adjusted to pH 4.2) | 505.9 ± 103.1 | =0.02 |

* statistically significant

These results indicate that a statistically significant increase (23%) in hair growth was obtained in this experiment.

## Examples

The invention is illustrated by the following examples.

### Example 1

This Example illustrates a lotion according to the invention which is suitable for topical application to the scalp in order to promote hair growth.

The lotion has the following formulation:

| | % w/w |
|---|---|
| N-acetyl glycine | 0.1 |
| ethanol | 99.995 |
| perfume | q.s. |

Example 2

This Example illustrates a hair tonic which is suitable for application to hair or scalp.

| | % w/w |
|---|---|
| N-acetyl alanine | 0.8 |
| ethanol | 50 |
| water | 49 |
| perfume | q.s. |

Example 3

This Example also illustrates a lotion which is suitable for topical application to the scalp. The lotion has the following formulation:

| | % w/w |
|---|---|
| N-acetyl valine | 1.5 |
| propan-2-ol | 10 |
| ethanol | 88.5 |
| perfume | q.s. |

Example 4

This Example also illustrates a hair tonic which is suitable for application to hair or scalp. The hair tonic has the following formulation:

| | % w/w |
|---|---|
| N-acetyl leucine | 0.2 |
| ethanol | 40 |
| water | 59.80 |
| perfume | q.s. |

Examples 5 to 8

The following formulations represent lotions which can be used topically in the treatment of bald or balding male or female heads.

| | % w/w | | | |
|---|---|---|---|---|
| | 5 | 6 | 7 | 8 |
| Hydroxyethyl cellulose | 0.4 | - | 0.4 | - |
| Absolute ethanol | 25 | 25 | 25 | 25 |
| Propane-1,2-diol | - | - | 38.4 | 38.4 |
| Butane-1,3-diol | 38.4 | 38.8 | - | - |
| Paramethyl benzoate | 0.2 | 0.2 | 0.2 | 0.2 |
| N-acetyl isoleucine | 5 | - | - | - |
| N-acetyl phenylalanine | - | 1 | - | - |
| N-acetyl tyrosine | - | - | 0.8 | - |
| N-acetyl proline | - | - | - | 0.6 |
| Perfume | 1 | 1 | 1 | 1 |
| Water | to 100 | 100 | 100 | 100 |

Examples 9 to 12

The following formulations represent creams which can be used in the treatment of baldness.

| | % w/w | | | |
|---|---|---|---|---|
| | 9 | 10 | 11 | 12 |
| Cetyl alcohol polyoxyethylene (10) | 4 | 4 | 4 | 4 |
| Cetyl alcohol | 4 | 4 | 4 | 4 |
| Mineral oil | 4 | 2 | - | - |
| Paraffin wax | - | 2 | 4 | - |
| Partial glyceride of palmitic and stearic acids | - | - | - | 4 |
| N-acetyl hydroxyproline | 2 | - | - | - |
| N-acetyl serine | - | 1.5 | - | - |
| N-acetyl threonine | - | - | 2 | - |
| N-acetyl cysteine | - | - | - | 1 |
| Triethanolamine | 0.75 | 0.75 | 0.75 | 0.75 |
| Butane-1,3-diol | 3 | 3 | 3 | 3 |
| Xanthan gum | 0.3 | 0.3 | 0.3 | 0.3 |
| Preservative | 0.4 | 0.4 | 0.4 | 0.4 |
| Perfume | q.s. | q.s. | q.s. | q.s. |
| Water | to 100 | 100 | 100 | 100 |

Example 13

This Example illustrates a water-in-oil high internal phase emulsion containing an ester according to the invention.

The emulsion consisted of 10% by volume oily phase and 90% by weight aqueous phase.
The oily phase and the aqueous phase had the following constitution:

| | % w/w |
|---|---|
| Oily phase | |
| Sorbitan monooleate | 20 |
| Quaternium-18 hectorite | 5 |
| Liquid paraffin | 75 |
| Aqueous phase | |
| N-acetyl cystine | 0.5 |
| Xanthan gum | 1 |
| Preservative | 0.3 |
| Perfume | q.s. |
| Sodium chloride (1% w/w solution) | to 100 |

The emulsion was prepared by taking 10 parts by volume of the oily phase and to it adding slowly with stirring 90 parts by volume of the aqueous phase.

The high internal phase water-in-oil emulsion so formed can be applied topically to the scalp, to improve hair growth and regrowth.

methyl
ethyl
propyl
iso-propyl
butyl
iso-butyl
n-valeryl
iso-valeryl
n-caproyl
n-heptyl
n-caprylyl
n-capryl
lauryl
myristyl
palmityl
stearyl, and
arachidyl.
and the $C_{10-22}$ alkenyl groups:
linoleyl
linolenyl
γ-linolenyl
arachidonyl, and
columbinyl.

Further examples of the grouping (24) also include hydroxyalkyl groups having from 1 to 22 carbon atoms, such as:
hydroxymethyl
2-hydroxyethyl
2-hydroxy-n-propyl
3-hydroxy-n-propyl
2-hydroxy-n-butyl
3-hydroxy-n-butyl

Example 16

This Example also illustrates a lotion which is suitable for topical application to the scalp. The lotion has the following formulation:

| | % w/w |
|---|---|
| N-acetyl aspartic acid | 1.5 |
| propan-2-ol | 10 |
| ethanol | 88.5 |
| perfume | q.s. |

Example 17

This Example also illustrates a hair tonic which is suitable for application to hair or scalp. The hair tonic has the following formulation:

| | % w/w |
|---|---|
| N-acetyl glutamic acid | 0.2 |
| ethanol | 40 |
| water | 59.80 |
| perfume | q.s. |

| Example 18 | |
|---|---|
| | % w/w |
| Monoethanolamine lauryl sulphate : [100% AD] | 20 |
| JAGUAR C13S | 3 |
| BRIPHOS 03D | 1.7 |
| Coconut diethanolamide | 5 |
| N-lauroyl glycine | 1 |
| Zinc gluconate | 3 |
| Perfume | q.s. |
| Water | to 100 |
| pH adjusted to 6.5 | |

| Example 19 | |
|---|---|
| | % w/w |
| Sodium lauryl ether sulphate (3 EO) : [100% AD] | 12 |
| JAGUAR C13S | 0.3 |
| BRIPHOS 03D | 1 |
| N-palmitoyl glycine | 2 |
| Sodium chloride | 4 |
| Perfume | q.s. |
| Water | to 100 |
| pH adjusted to 6.5 | |

| Example 20 | |
|---|---|
| | % w/w |
| Sodium lauryl ether sulphate ( 2 EO) [100% AD] | 12 |
| POLYMER JR400 | 3 |
| BRIPHOS 03D | 1 |
| Opacifier | 9 |
| N-myristoyl glycine | 5 |
| Perfume | q.s. |
| Water | to 100 |
| pH adjusted to 6.5 | |

Examples 21

This example illustrates a powder composition according to the invention which can be applied topically to the scalp.

| | % w/w |
|---|---|
| Chemically modified starch | 5 |
| Chemically modified cellulose | - |
| Boric acid | 10 |
| Zinc oxide | 5 |
| N-lauroyl hydroxyproline | 3 |
| Minoxidil glucuronide | 5 |
| Perfume | q.s. |
| Chalk | 10 |
| Talc | to 100 |

Example 22

The following example illustrates a lotion according to the invention which can be applied topically to the scalp to prevent hair loss and stimulate hair regrowth.

| | % w/w |
|---|---|
| N-palmitoyl aspastic acid | 7 |
| Minoxidil | 0.2 |
| ethanol | 16 |
| citric acid | 1.05 |
| water | to 100 |
| pH adjusted to 4.2 with sodium hydroxide | |

Examples 23 & 24

These examples illustrate hair tonics which are suitable for application to the hair and scalp. The hair tonics had the following formulation:

| | % w/w | |
|---|---|---|
| | 26 | 27 |
| N-octanoyl glycine | 2 | - |
| N-octanoyl hydroxyproline | - | 3 |
| ethanol | 50 | 50 |
| water | 48 | 47 |
| perfume | q.s. | q.s. |

Example 25

This example illustrates a microgel which is suitable for topical application to hair or scalp. The gel had the following formulation:

| | | % w/w |
|---|---|---|
| A. | Polyoxyethylene (10) oleyl ether | 14.5 |
| | Polyoxyethylene fatty glyceride | 14.5 |
| | Light liquid petroleum | 13.7 |
| | Propylene glycol | 7.6 |
| | Sorbitol | 5.9 |
| | N-hexanoyl glycine | 4 |
| B. | Perfume | q.s. |
| C. | Water | to 100 |

This microgel was prepared by heating part A to 90°C and part C to 95°C and then adding part C to part A with stirring. Part B was then added at 70°C and the final mixture cooled and poured into jars at 55°C to 60°C. On further cooling, a gel was formed.

Example 26

This example illustrates a shampoo which is suitable for topical application to hair in order to cleanse it, at the same time delivering an inhibitor to the scalp to enhance hair growth or regrowth.

The shampoo had the following formulation:

| | | % w/w |
|---|---|---|
| Triethanolamine lauryl sulphate | | 16.8 |
| Coconut diethanolamide | | 3.0 |
| Hydroxypropylmethylcellulose (1) | | 0.25 |
| Corn syrup (80% solids) (2) | | 20.5 |
| Dimethylpolysiloxane (3) | | 1.0 |
| Cationic cellulose (4) | | 0.5 |
| Ethyl alcohol (SDA 40) | | 9.0 |
| Vinyl carboxy polymer (5) | | 0.75 |
| N-hexanoyl aspastic acid | | 1 |
| Perfume, colour, preservative | | q.s. |
| Water | | to 100 |
| Acid or base | to pH: | 6.5 |

1 - Methocel E4M (Dow Chemical)
2 - 42 Dextrose equivalent (Staley 1300)
3 - 60,000 centistokes (Viscasil, GEC)
4 - Polymer JR 400
5 - Carbopol 941 (BF Goodrich)

## Examples 27 to 28

The following formulations represent lotions which can be used topically in the treatment of bald or balding male or female heads.

| | % w/w | |
|---|---|---|
| | 27 | 28 |
| Hydroxyethyl cellulose | 0.4 | - |
| Absolute ethanol | 25 | 25 |
| Propane-1,2-diol | - | - |
| Butane-1,3-diol | 38.4 | 38.8 |
| Paramethyl benzoate | 0.2 | 0.2 |
| N-acetyl glycine | 5 | - |
| N-acetyl hydroxyproline | - | 1 |
| Perfume | 1 | 1 |
| Water | to 100 | 100 |

**Claims**

1. A composition suitable for topical application to mammalian skin and hair for inducing, maintaining or increasing hair growth, which comprises:
i. an effective amount of from 0.001 to 99% by weight of a hair growth promoter chosen from:
N-acylated neutral or acidic amino acids, or salts thereof, in which the acyl group has from 1 to 18 carbon atoms; and
ii. from 1 to 99.999% by weight of a cosmetically acceptable vehicle for the hair growth promoter;
the total amount of the hair growth promoter present in the composition being sufficient to increase hair growth in the rat, when said composition is applied topically thereto over a period of no more than 3 months, by at least 10% more than that obtainable using a control composition from which the said promoter has been omitted, in accordance with the Rat Hair Growth Test.
2. A composition according to claim 1, in which the hair growth promoter is chosen from:
N-acetyl glycine
N-acetyl hydroxyproline
N-acetyl aspartic acid, and
mixtures thereof.
3. A composition according to any preceding claim, in which the hair growth promoter forms from 0.01 to 20% by weight of the composition.
4. A composition according to any preceding claim, in which the vehicle acts as a preservative.
5. A composition according to any preceding claim which further comprises an activity enhancer.
6. A composition according to Claim 5, in which the vehicle acts as an activity enhancer.
7. A composition according to Claim 5 or 6, in which the activity enhancer is another hair growth stimulant.
8. A composition according to claim 7, in which the other hair growth stimulant is minoxidil.
9. A composition according to Claim 5, in which the activity enhancer is a penetration enhancer.
10. A composition according to Claim 9, in which the penetration enhancer is a surface active agent.
11. A composition according to Claim 6, in which the activity enhancer is a cationic polymer.
12. A composition according to any preceding claim which has a pH value of from 2 to <7.
13. A composition according to any preceding claim, which is a shampoo or hair conditioner.

European
Patent Office

# EUROPEAN SEARCH REPORT

Application Number

**EP 90 30 8960**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 327 263 (PROCTOR)<br>* Claims 1-4,13-14,30 * | 1,3-10,13 | A 61 K 7/06<br>A 61 K 7/48 |
| X | DE-A-1 910 561 (HERTEN)<br>* Example; claims 1,7-10 * | 1,3,5-7,9,13 | |
| X | CHEMICAL ABSTRACTS, vol. 84, 1974, page 327, abstract no. 68351t, Columbus, Ohio, US;<br>& JP-A-74 00 451 (AJINOMOTO CO., LTD) 05-01-1974<br>* Abstract * | 1,3,7,13 | |
| X | EP-A-0 308 278 (LES ETABLISSEMENTS GIVAUDAN LAVIROTTE & CIE)<br>* Whole document * | 1-3,5-6,9-10,12-13 | |
| X | FR-A-2 503 151 (MORELLE et al.)<br>* Whole document * | 1,3,5-6,9-10,13 | |
| X | FR-A-2 192 795 (Dr. KARL THOMAE GmbH)<br>* Whole document * | 1-3,5-6,9-10,13 | |
| | | | TECHNICAL FIELDS SEARCHED (Int. Cl.5) |
| A | GB-A-2 183 632 (KEMYOS BIO MEDICAL RESEARCH)<br>* Examples 5,E; claims * | 1-13 | A 61 K |
| A,P | EP-A-0 362 030 (NORCHIM)<br>* Whole document * | 1-13 | |
| X | FR-A-1 167 188 (LUCIANI)<br>* Whole document * | 1,3-4,13 | |

The present search report has been drawn up for all claims

| Place of search | Date of completion of search | Examiner |
|---|---|---|
| The Hague | 28 November 90 | FISCHER J.P. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document